# EUROPÄISCHE PATENTSCHRIFT

(11) **EP 1 393 770 B1**
(45) Veröffentlichungstag und Bekanntmachung des Hinweises auf die Patenterteilung: **26.10.2005**
(21) Anmeldenummer: 03017807.3
(22) Anmeldetag: 05.08.2003
(51) Int. Cl.: A61M 25/00, A61M 39/02, A61M 1/16

(54) **Medizinisches Arbeitsmittel**
Medical article
Article médical

(30) Priorität: 29.08.2002 DE 10239744
(43) Veröffentlichungstag der Anmeldung: 03.03.2004
(73) Patentinhaber: RAUMEDIC AG, 95233 Helmbrechts (DE)
(72) Erfinder: Gropp, Friedrich, 95111 Rehau (DE); Stendel, Rüdiger, 14163 Berlin (DE)
(74) Vertreter: Hofmann, Matthias

(56) Entgegenhaltungen:
- EP-A- 1 145 731
- WO-A-90/12611
- WO-A-95/16405
- WO-A-96/14889
- US-A- 6 030 358
- US-A1- 2002 115 956
- US-B1- 6 198 966

## Beschreibung

Die Erfindung betrifft eine Vorrichtung, die vorzugsweise die kontinuierliche Abgabe von Wirkstoffen und die Messung ihrer Konzentration erlaubt.

Ein bekanntes Problem in der Medizin ist der genau dosierte und/oder kontinuierlich anwendbare Wirkstofftransport, beispielsweise wenn Tumoren oder Entzündungsherde in oder an Organen zu behandeln sind.

Nach dem aktuellen Stand der Technik wird für eine fallweise Medikation der Wirkstoff in einer speziellen, galenischen Zubereitung, oral, intramuskulär, subkutan, intravenös usw verabreicht.

Dazu wendet man in der Regel die folgenden drei Verfahren an:
1. Die sogenannte systemische Applikation
   Hierbei erreicht man durch Verteilung der Wirkstoffe im ganzen Körper den Wirkort in einer lediglich grob schätzbaren Dosierung.
2. Die regionale Applikation
   Die Gabe des Wirkstoffes erfolgt in Arterien, deren Versorgungsbereich das Zielgebiet beinhaltet.
3. Die lokale Applikation
   Dazu gehören die Anwendungen von Gels oder Salben, weiterhin die Spülungen von Tumorhöhlungen oder von Entzündungsherden, auch die Injektionen in Gelenke und andere.

Bei all diesen Methoden wird der Wirkstoff in der Regel mit Hilfsstoffen kombiniert, welche den Transport an den Wirkort ermöglichen oder verbessern und die Dosierung in gewissen Grenzen steuern helfen.

Diese Verfahren belasten allerdings fast immer die Bereiche um den Wirkort und oft sogar den ganzen Körper, so dass das Auftreten unerwünschter Nebenwirkungen die Applikation in der notwendigen Dosierung meist unmöglich machen.

Auf Grund der Verteilungsverluste muss zudem die Dosierung in der Regel von Anfang an höher gewählt werden, was die Nebenwirkungen weiter erhöht. Außerdem kann die tatsächliche Wirkstoffkonzentration am Wirkort nur abgeschätzt werden, da zu viele, meist unvorhersehbare, Faktoren einwirken.
Speziell bei der Tumorbekämpfung ergeben sich daher langwierige und für den Patienten risikoreiche Therapien, um eine Optimierung der Medikation zu erreichen; trotzdem ist das Ergebnis mit diesen Verfahren nicht selten unbefriedigend.

Geht es an Stelle einer fallweisen Medikation um eine Wirkstoffgabe über längere Zeiträume, werden z. B. Infusionen in den Blutkreislauf infundiert und in der Regel nach dem Körpergewicht und der Körperoberfläche des Patienten dosiert. Soll an einem relativ kleinen Wirkort, beispielsweise einem Entzündungsherd, eine Wirkung erreicht werden, so versuchte man mit speziellen Kathetern diesen Bereich anzugehen, um einen Wirkstoffbolus über den Katheter zu injizieren.
Die Wirkung ist dann zwar weitgehend auf diesen Bereich beschränkt, die Dosierung ist in der Folge jedoch schlecht zu steuern, so dass dieser Weg nur im Falle einer relativen Ungefährlichkeit des Wirkstoffes angewendet werden kann.

Instrumente, um Wirkstoffe zu applizieren, sind: Mit Injektionsnadeln verbundene Spritzen, manuelle oder mit Pumpsystemen verbundene Infusionssysteme, subkutan implantierbare Portsysteme, implantierbare Kapseln oder mit Wirkstoffen getränkte Hilfsstoffe usw.
Die Bestimmung der tatsächlich im Gewebe, also dem Wirkort, erreichten Wirkstoffkonzentrationen, ist bei Menschen in der Regel nicht möglich, sie lässt sich nur auf Grund von Tierversuchen abschätzen.

Zusammengefasst haben die genannten Verfahren hauptsächlich folgende Nachteile:
- Der Wirkstoff wird immer an Trägersubstanzen für Transport, Dosierung oder Anbindung gekoppelt sein
- Eine exakte Dosierung ist über längere Zeiträume nicht möglich
- Keine gleichzeitige Applikation des Werkstoffes und Messung seiner Konzentration am Wirkort möglich
- Wirkstoffkonzentration am Wirkort abhängig von Perfusion und Metabolismus
- Keine kontinuierliche, gesteuerte und kontrollierte Wirkstoffabgabe möglich

Aus der US 6 030 358 A ist eine Kathetereinheit mit einem großlumigen Katheter bekannt, in den axial ein kleinlumiger Katheter eingebracht ist. Das distale Ende des kleinlumigen Katheters mündet in den großlumigen Katheter aus. Der großlumige Katheter weist eine mikroporöse Außenwand auf.

Die Aufgabe der Erfindung stellte sich darin, eine Vorrichtung zur Verfügung zu stellen, die im Wesentlichen über folgende vorteilhafte Eigenschaften verfügt:
- Möglichkeit der punktuellen, direkten Wirkstoffgabe und damit Erzielen minimaler Verbrauchsmengen und Nebenwirkungen
- Der Wirkstoff lässt sich in reiner Form applizieren, Träger- und Mittlersubstanzen, sowie Volumenbelastungen entfallen bei der Anwendung
- Patientenbelastungen werden wesentlich gesenkt, da keine Wirkstoffkonzentrationen im Rahmen des Wirkstofftransportes im gesunden Gewebe auftreten
- Gleichzeitig mit der Applikation des Wirkstoffes kann dessen Konzentration gemessen werden

Die Aufgabe konnte überraschenderweise gelöst werden durch eine Vorrichtung gemäß Anspruch 1. Hierbei erfolgt die kontinuierliche Abgabe von Wirkstoffen über einen semipermeablen Bereich in einem dreilumigen Katheter, wobei gleichzeitig über die kontinuierliche oder quasi-kontinuierliche Messung des Verbrauches mittels Brechungsindex, Potentialmessungen, IR- oder UV-Spektroskopie usw. exakte Rückschlüsse auf die applizierte Wirkstoffmenge möglich sind. Nachfolgend soll nun die Erfindung näher erläutert werden:

Die erfindungsgemäße Vorrichtung dient zur Applikation von Wirkstoffen, z. B. verschiedener Chemotherapeutika in parenchymatösen Organen, insbesondere im Sinne der Tumorbehandlung und lokalen Behandlung von Infektionen und gleichzeitiger Messung der Wirkstoffkonzentration.
Die Vorrichtung besteht aus einer zentralen Funktionseinheit in Form eines mehrlumigen Katheters und verschiedener Peripherieeinheiten, insbesondere Pumpen, Vorrats- und Auffanggefäße und Möglichkeiten zur Wirkstoffanalyse.
Die Behandlung des gewünschten Gewebeteils erfolgt dergestalt, dass über einen Katheter, der zum Wirkort über eine Führungshülse oder einen Führungsdraht gelegt wird, proximal ein Reservoir mit definierter Wirkstoffkonzentration gekoppelt ist, so dass der Wirkstoff ohne die Passage durch den Körper direkt zum Wirkort geleitet wird. Der Katheter ist an seinem semipermeablen Bereich so gestaltet, dass es möglich ist, nur die Molekülgrößen des oder der Wirksubstanzen in das umgebende Gewebe abzugeben, ohne dass Körpersubstanzen in den Katheter gelangen. Die Dosierung der Wirksubstanzen kann kontinuierlich, durch den Volumenstrom oder die Veränderung der Wirkstoffkonzentration erfolgen oder diskontinuierlich durch das Füllen des Katheterlumens mit Wirkstoff und die Abgabe des Wirkstoffes aus der stehenden Säule des Katheterlumens.
Die Arbeitsweise ist auch wechselseitig zu handhaben.
Die Abgabegeschwindigkeit des Wirkstoffes ist abhängig von dessen Konzentrationsgefälle zwischen Katheterlumen und Katheterumgebung.
Mit steigender Wirkstoffkonzentration der Katheterumgebung ist der Zustrom von Wirkstoff aus dem Katheterlumen reduziert, bis ein Gleichgewicht erreicht ist. So ist zielgerichtet der Gewebebereich versorgt, in dem eine andauernde und genau dosierte Wirkung erreicht werden muss. Die Konzentration des Wirkstoffes kann am Reservoir, der Pumpe oder am Katheterrücklauf gemessen werden, damit ist jederzeit der Verbrauch ermittelbar und Korrekturen sind entsprechend abzuleiten.
Zur Gabe von massiven Initialwirkstoffmengen ist ein, im Katheter integriertes, Lumen, unabhängig von den bereits vorhandenen, gestaltet, das den direkten Zulauf des Wirkstoffes sichert und zusätzlich die Probenahme zur Analyse ermöglicht.

Die direkte Wirkstoffabgabe erlaubt minimierte Verbrauchsmengen und minimierte Nebenwirkungen.

Zusammengefasst hat die erfindungsgemäße Vorrichtung folgende, hoch erwünschte Vorteile:
- Die Dosierung ist abhängig vom Konzentrationsgradienten in den Schlauchsegmenten und der Gewebeumgebung (Eigenregulation)
- Der Wirkstoffübergang erfolgt am Wirkort
- Kleinste Mengen Wirkstoff können über die Auswahl des richtigen, semipermeablen Materials selektiv appliziert werden
- Die Vorrichtung ist in ein geschlossenes System integrierbar, bis hin zu Kreisläufen, in denen keine Wirkstoffverluste auftreten.

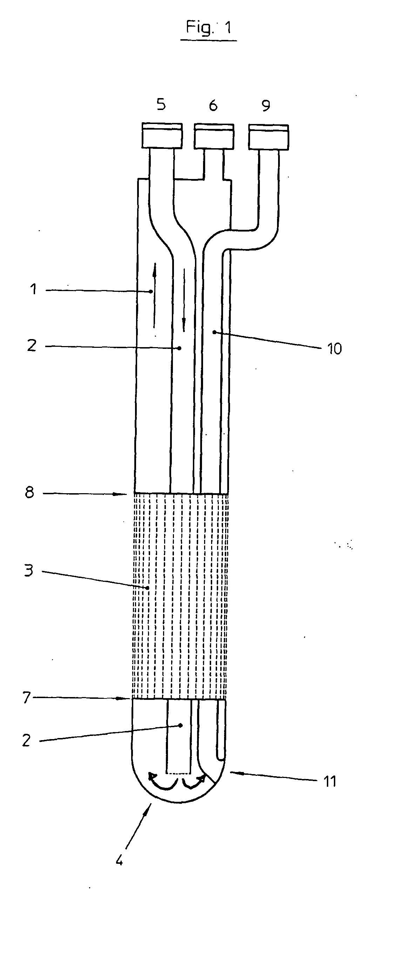

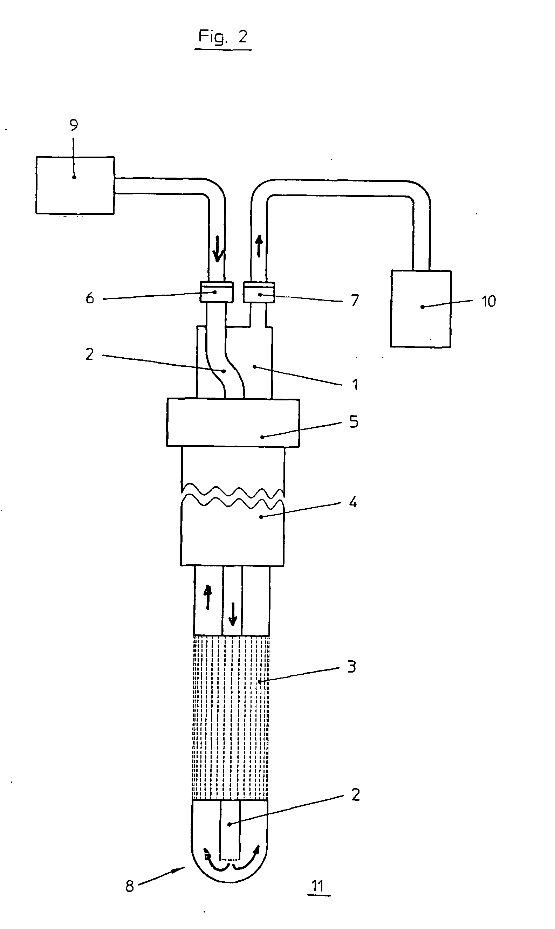

Nachfolgend wird eine mögliche Ausführungsform der erfindungsgemäßen Vorrichtung anhand von Figur 1 im Detail beschrieben. Figur 2 zeigt einen Katheter aus dem Stand der Technik.

### zu Figur 1:

Figur 1 zeigt einen dreilumigen Katheter, der sowohl für die Verabreichung kontinuierlicher Wirkstoffgaben, als auch für die gleichzeitige Messung der verabreichten Wirkstoffmengen geeignet ist, zusätzlich noch die Möglichkeit für die Verabreichung einmaliger, hoher Wirkstoffkonzentrationen bietet.
Dazu ist der Katheter folgendermaßen aufgebaut:

In einem großlumigen Katheter 1 ist axial ein kleinlumiger Katheter 2 eingebracht. Beide verfügen am einen Ende über die Anschlussstücke 5 und 6, am anderen Ende ragt der kleinlumige Katheter 2 über den großlumigen Katheter 1 hinaus. In diesem Bereich ist der kleinlumige Katheter über den gesamten Umfang mit Schlauchsegmenten 3 besetzt, die aus semipermeablem Material bestehen und die fest mit der Oberfläche des kleinlumigen Katheters 2 verbunden sind.
Am distalen Ende 7 werden die offenen Enden der Schlauchsegmente 3 und des kleinlumigen Katheters 2 nahtlos von einer atraumatischen Spitze 4 aufgenommen, am proximalen Ende der Schlauchsegmente 8 ragen diese nahtlos in den großlumigen Katheter 1 hinein.

Beide Katheter 1 und 2 lassen sich getrennt an manuelle und maschinelle Zu- und Ableitungssysteme über die Verbindungsanschlüsse 5 und 6 dicht ankoppeln.
Ein drittes Lumen 10, mit Anschluss für eine Spritze 9 und Öffnung 11 an der Katheterspitze 4 ist zur Probenahme und zur massiven Wirkstoffgabe geeignet.
Die Außendurchmesser der Spitze 4, der Schlauchsegmente 3 und des großlumigen Katheters 1 sind einheitlich.

Die beschriebene Konfiguration gewährleistet den Wirkstofffluss über den kleinlumigen Katheter 2 in die Spitze 4, dort ändert sich die Fließrichtung und aus dem Hohlraum der Spitze 4 strömt der Wirkstoff in die Schlauchsegmente 3, deren semipermeables Material Wirkstoffe mit einem Molekulargewicht von 280 g pro Mol in die Umgebung passieren lässt. Nicht abgegebener Wirkstoff durchfließt die Schlauchsegmente 3 und strömt in den großlumigen Katheter 1, um gesammelt und erneut dem Fließzyklus beigegeben zu werden.
Ausführungen mit üblichen Applikationshilfen oder mit manuellen und maschinellen Schwerkraft- oder Pumpsystemen sind abhängig vom konkreten Anwendungsfall. Die Materialien, aus denen die Schlauchsegmente 3 gefertigt werden, lassen die Permeation verschiedenartigster Molekulargewichte vor, wobei erfindungsgemäß die Hauptanwendung in den nieder- und mittelmolekularen Bereichen liegen.

### zu Figur 2:

Figur 2 beschreibt ein Katheter einschließlich einiger Peripherieeinheiten.
Die Kathetereinheit 11 besteht aus einem großlumigen Katheter 1, in welchen ein kleinlumiger Katheter 2, der abschnittsweise auf seinem Umfang Schlauchsegmente 3 aus semipermeablem Material trägt, axial eingebracht ist.
Die Platzierung am Wirkort erfolgt gemäß dem Stand der Technik mittels atraumatischem Mandrin und Hülse 4, in die schließlich - nach Entnahme des Mandrins - die Kathetereinheit 11 eingeführt wird.
Proximal ist die Kathetereinheit 11 mit dem Lumenanschluss 6 des kleinlumigen Katheters 2 an eine Spritzenpumpe 9 mit Wirkstoffspritze gekoppelt und gefüllt. Indem Wirkstoff durch den kleinlumigen Katheter 2 in die Katheterspitze 8 gelangt, erfolgt dort die Befüllung der Schlauchsegmente 3 aus semipermeablem Material.

Diese Flüssigkeitsmengen sammeln sich im großlumigen Katheter 1 und fließen über dessen Lumenanschluss 7 in einen Auffangbeutel 10. Nach dem Befüllen des Systems wird die Hülse 4 über die erforderliche Länge zurückgezogen, die Kathetereinheit 11 über das Ventil 5 fixiert und damit die Schlauchsegmente 3 mit dem umgebenden Gewebe in Verbindung gebracht.
Mit der kontinuierlichen Arbeit der Spritzenpumpe 9 erfolgt die permanente Durchströmung der Schlauchsegmente 3 mit Wirkstoff und dessen Diffusion in das Gewebe.

## Patentansprüche

1. Vorrichtung, zusammengesetzt aus einer zentralen, mehrlumigen Kathetereinheit und verschiedenen Peripherieeinheiten,
wobei
- die zentrale Kathetereinheit aus einem großlumigen Katheter (1) besteht, in den axial ein kleinlumiger Katheter (2) eingebracht ist, wobei beide am einen Ende über Anschlussstücke (5 und 6) verfügen, der kleinlumige Katheter am anderen Ende über den großlumigen Katheter hinausragt und in diesem Bereich über den gesamten Umfang mit Schlauchsegmenten (3) besetzt ist, die aus semipermeablem Material bestehen, die fest mit der Oberfläche des kleinlumigen Katheters (2) verbunden sind und die am distalen Ende mit einer Spitze (4) gekoppelt sind, welche die offenen Enden der Schlauchsegmente und des kleinlumigen Katheters aufnimmt, wobei die Schlauchsegmente mit ihrem proximalen Ende in den großlumigen Katheter hineinragen,
- die Kathetereinheit ein drittes Lumen (10) aufweist, welches einen Anschluß für eine Spritze und eine Öffnung an der Katheterspitze aufweist.

2. Vorrichtung nach Anspruch 1, **dadurch gekennzeichnet, dass** die Peripherieeinheiten bestehen aus Pumpen- und/oder Analyse-Geräten und/oder Vorratsgefäßen und/oder Auffanggefäßen und/oder EDV-Geräten.

3. Vorrichtung nach Anspruch 2, **dadurch gekennzeichnet, dass** die semipermeablen Schlauchsegmente in ihrer Trenngrenze so ausgeführt sind, dass gezielt einzelne Wirkstoffe und/oder Wirkstoffkombinationen permeieren.

## Claims

1. A device comprised of a central, multi-lumen catheter unit and various peripheral units,
wherein
- the central catheter unit is composed of a large lumen catheter (1), in which a small lumen catheter (2) is axially integrated, both having connecting pieces (5 and 6) at one end and the small lumen catheter, at the other end, projecting over the large lumen catheter and, in this area and over its entire circumference, being equipped with tube segments (3) of semi-permeable material which are fixedly connected to the surface of the small-lumen catheter (2) and, at their distal end, are coupled with a tip (4) which accommodates the open ends of the tube segments and of the small lumen catheter, with the tube segments, by their proximal end, projecting into the large lumen catheter;
- the catheter unit has a third lumen (10) which comprises a connection for a syringe and an opening at the tip of the catheter.

2. A device according to claim 1, **characterized in that** the peripheral units are comprised of pumps and/or analyzers and/or storage vessels and/or collecting vessels and/or EDP equipment.

3. A device according to claim 2, **characterized in that** the semi-permeable tube segments, at their interfaces, are designed such that individual agents and/or combinations of agents permeate selectively.

## Revendications

1. Dispositif comportant une unité de cathéters centrale à plusieurs lumières et différentes unités périphériques,
dans lequel
- l'unité de cathéters centrale est formée par un cathéter (1) avec une grande lumière, dans lequel est enfiché axialement un cathéter (2) avec une petite lumière, tous deux comportant des embouts de raccordement (5 et 6) au niveau d'une extrémité, le cathéter à petite lumière s'avançant en saillie au niveau de son autre extrémité en dehors du cathéter à grande lumière (1) et est occupé dans cette zone sur toute la surface périphérique de segments de tuyaux (3) qui sont réalisés en matériau semi-perméable, qui sont reliés fermement à la surface du cathéter à petite lumière (2) et qui sont couplés au niveau de l'extrémité distale à une pointe (4), qui reçoit les extrémités ouvertes des segments de tuyaux et du cathéter à petite lumière, les segments de tuyaux pénétrant avec leur extrémité proximale dans le cathéter à grande lumière,
- l'unité de cathéters comporte une troisième lumière (10), qui comporte un raccordement pour une seringue et une ouverture au niveau de la pointe du cathéter.

2. Dispositif selon la revendication 1, **caractérisé en ce que** les unités périphériques sont formées par des pompes et/ou des appareils pour analyse et/ou des réservoirs et/ou des collecteurs et/ou des appareils informatiques.

3. Dispositif selon la revendication 2, **caractérisé en ce que** les segments de tuyaux semi-perméables sont réalisés dans leur limite de séparation de manière à laisser traverser, de façon ciblée, des réactifs isolés et/ou des combinaisons de réactifs.
